(19) Europäisches Patentamt
European Patent Office
Office européen des brevets

(11) Veröffentlichungsnummer: **0 002 231**
**A1**

# (12) EUROPÄISCHE PATENTANMELDUNG

(21) Anmeldenummer: **78101431.1**

(22) Anmeldetag: **22.11.78**

(51) Int. Cl.[2]: C 07 D 211/90
C 07 D 401 04, A 61 K 31/44

(30) Priorität: **03.12.77 DE 2753946**

(43) Veröffentlichungstag der Anmeldung:
**13.06.79 Patentblatt 79/12**

(84) Benannte Vertragsstaaten:
**BE CH DE FR GB NL SE**

(71) Anmelder: **Bayer Aktiengesellschaft**
**Zentralbereich Patente,Marken und Lizenzen Bayerwerk**
**D-5090 Leverkusen 1(DE)**

(72) Erfinder: **Bossert, Friedrich, Dr.**
**Claudiusweg 7**
**D-5600 Wuppertal 1(DE)**

(72) Erfinder: **Heise, Arend, Dr.**
**Moebeck 50**
**D-5600 Wuppertal 1(DE)**

(72) Erfinder: **Kazda, Stanislav, Dr.**
**Pahlkestrasse 55 N**
**D-5600 Wuppertal 1(DE)**

(72) Erfinder: **Klauke, Erich, Dr.**
**Eichendorffweg 8**
**D-5068 Odenthal(DE)**

(72) Erfinder: **Stoepel, Kurt, Dr.**
**In den Birken 69**
**D-5600 Wuppertal 1(DE)**

(54) **1-N-Aryl-1,4-dihydropyridine, Verfahren zu ihrer Herstellung und ihre Verwendung als Arzneimittel.**

(57) 1-N-Aryl-1,4-dihydropyridine der Formel

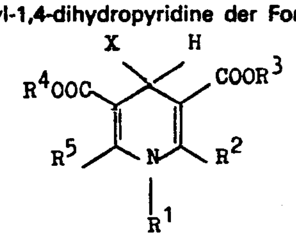

in welcher

$R^1$ für einen gegebenenfalls substituierten Arylrest steht,

$R^2$ und $R^5$ für gegebenenfalls substituiertes Alkyl oder Alkoxyalkyl stehen,

$R^3$ und $R^4$ gleich oder verschieden, jeweils für gegebenenfalls substituiertes Alkyl, Alkenyl oder Alkinyl stehen,

X für einen gegebenenfalls substituierten Alkyl- oder Arylrest steht oder für gegebenenfalls substituiertes Aralkyl, Styryl, Cycloalkyl, Cycloalkenyl, Chinolyl, Isochinolyl, Pyridyl, Pyrimidyl, Furyl, Thienyl oder Pyrryl steht,

mehrere Verfahren zu ihrer Herstellung sowie ihre Verwendung als Arzneimittel, insbesondere als kreislaufbeeinflussende Mittel.

EP 0 002 231 A1

Croydon Printing Company Ltd.

BAYER AKTIENGESELLSCHAFT
Zentralbereich
Patente, Marken und Lizenzen

5090 Leverkusen, Bayerwerk
Ib(Pha)
KS/k

BEZEICHNUNG GEÄNDERT
siehe Titelseite

1-N-Aryl-1,4-dihydropyridine und ihre Verwendung als Arzneimittel

Die vorliegende Erfindung betrifft neue 1-N-Aryl-1,4-dihydropyridine, mehrere Verfahren zu ihrer Herstellung sowie ihre Verwendung als Arzneimittel, insbesondere als kreislaufbeeinflussende Mittel.

Es ist bereits bekannt geworden, daß 1,4-Dihydropyridine interessante pharmakologische Eigenschaften besitzen (F. Bossert und W. Vater, Naturwissenschaften, 58, 578 (1971)).

Die Erfindung betrifft neue 1-N-Aryl-1,4-dihydropyridine der allgemeinen Formel I

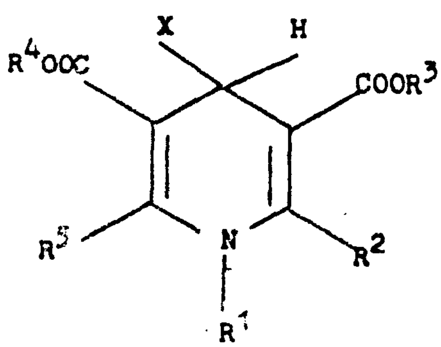

I

in welcher

$R^1$ für einen Arylrest steht, der gegebenenfalls durch 1, 2 oder 3 gleiche oder verschiedene Substituenten aus der Gruppe Halogen, Hydroxy, Carboxy, Trifluormethyl, Nitro, Cyano, Alkyl, Alkoxy oder den Rest $SO_n-R'$, wobei n für 0, 1 oder 2 steht und R' gegebenenfalls substituierten Alkyl bedeutet, substituiert ist, wobei die Alkyl- und Alkoxyreste gegebenenfalls substituiert sind und/oder in der Kette durch Sauerstoff, Stickstoff oder eine CO-Gruppierung unterbrochen sind,

$R^2$ und $R^5$ für gegebenenfalls substituiertes Alkyl oder Alkoxyalkyl stehen,

$R^3$ und $R^4$ gleich oder verschieden sind und jeweils für gegebenenfalls substituiertes Alkyl, Alkenyl oder Alkinyl stehen, wobei die Alkyl- und Alkenyl-Reste gegebenenfalls durch Sauerstoff oder Stickstoff in der Kette unterbrochen sind, und

X für einen gegebenenfalls substituierten Alkylrest steht oder für einen Arylrest steht, der gegebenenfalls durch 1, 2 oder 3 gleiche oder verschiedene Substituenten aus der Gruppe Nitro, Cyano, Azido, Halogen, Trifluormethyl, Trifluormethoxy, Phenyl, Hydroxy, Amino, Alkyl, Alkoxy, Alkoxycarbonyl, Acyloxy, Acylamino, Monoalkylamino, Dialkylamino und $SO_n$-Alkyl substituiert ist, wobei n 0, 1 oder 2 bedeutet
oder
für gegebenenfalls substituiertes Aralkyl ... Cyclo-

alkyl, Cycloalkenyl, Chinolyl, Isochinolyl, Pyridyl, Pyrimidyl, Furyl, Thienyl oder Pyrryl steht

sowie ihre pharmakologisch unbedenklichen Salze.

Es wurde gefunden, daß man die 1-N-Aryl-1,4-dihydropyridine der allgemeinen Formel I erhält, wenn man

a) ß-Ketocarbonsäureester der Formel II

$$R^2\text{-CO-CH}_2\text{-COOR}^3 \qquad (II)$$

in welcher

$R^2$ und $R^3$ die oben angegebene Bedeutung besitzen,

mit Aminen oder Formel III

$$H_2N\text{-}R^1 \qquad (III)$$

in welcher

$R^1$ die oben angegebene Bedeutung hat

oder deren Salzen gegebenenfalls nach Isolierung der hierbei entstehenden Enamine der Formel IV

$$\begin{array}{c} HN\text{-}R^1 \\ | \\ R^2\text{-C=CH-COOR}^3 \end{array}$$

in welcher

$R^1$, $R^2$ und $R^3$ die oben angegebene Bedeutung besitzen,

mit Yliden-Derivaten der Formel V

$$X-CH=\underset{COOR^4}{C}-\overset{O}{\overset{\|}{C}}-R^5 \qquad (V)$$

in welcher

X, $R^4$ und $R^5$ die oben angegebene Bedeutung besitzen,

gegebenenfalls in Gegenwart von inerten organischen Lösungsmitteln bei Temperaturen zwischen 20 und 150°C umsetzt,

oder

b) ß-Ketocarbonsäureester der Formel VI

$$R^5-CO-CH_2-COOR^4 \qquad (VI)$$

in welcher

$R^4$ und $R^5$ die oben angegebene Bedeutung besitzen,

mit Aminen der Formel III

$$H_2N-R^1 \qquad (III)$$

in welcher

$R^1$ die oben angegebene Bedeutung hat

oder deren Salzen gegebenenfalls nach Isolierung der hierbei entstehenden Enamine der Formel VII

$$\begin{matrix} \text{NH-}R_1 \\ R^5\text{-}\overset{|}{C}\text{=CH-COOR}^4 \end{matrix} \qquad \text{(VII)}$$

in welcher

$R^1$, $R^4$ und $R^5$ die oben angegebene Bedeutung besitzen,

mit Yliden-Derivaten der Formel VIII

$$\begin{matrix} \text{X-CH=C-CO-}R^2 \\ \overset{|}{\text{COOR}^3} \end{matrix} \qquad \text{(VIII)}$$

in welcher

$R^2$, $R^3$ und X die oben angegebene Bedeutung besitzen,

gegebenenfalls in Gegenwart von inerten organischen Lösungsmitteln bei Temperaturen zwischen 20 und 150°C umsetzt,

oder

c) 3-Ketocarbonsäureester der Formel VI

$$R^5\text{-CO-CH}_2\text{-COOR}^4 \qquad \text{(VI)}$$

in welcher

$R^4$ und $R^5$ die oben angegebene Bedeutung besitzen,

und Enamine der Formel IV

$$R^2-\overset{\overset{NH-R^1}{|}}{C}=CH-COOR^3 \qquad (IV)$$

in welcher

$R^1$, $R^2$ und $R^3$ die oben angegebene Bedeutung besitzen,

mit Aldehyden der Formel IX

$$X-CHO \qquad (IX)$$

in welcher

X die oben angegebene Bedeutung hat,

gegebenenfalls in Gegenwart von inerten organischen Lösungsmitteln bei Temperaturen zwischen 20 und 150°C umsetzt,

oder

d) ß-Ketocarbonsäureester der Formel II

$$R^2-CO-CH_2-COOR^3 \qquad (II)$$

in welcher

$R^2$ und $R^3$ die oben angegebene Bedeutung besitzen,

mit Enamine der Formel VII

$$R^5-\underset{}{\overset{NH-R^1}{C}}=CH-COOR^4 \qquad (VII)$$

in welcher

$R^1$, $R^4$ und $R^5$ die oben angegebene Bedeutung besitzen,

mit Aldehyden der Formel IX

$$X-CHO \qquad (IX)$$

in welcher

X die oben angegebene Bedeutung besitzt,

gegebenenfalls in Gegenwart von inerten organischen Lösungsmittel bei Temperaturen zwischen 20 und 150°C umsetzt,

oder

e) falls $R^2$ mit $R^5$ und $R^3$ mit $R^4$ identisch sind, zwei Teile β-Ketocarbonsäureester der Formel VI

$$R^5\text{-CO-CH}_2\text{-COOR}^4 \qquad (VI)$$

in welcher

$R^4$ und $R^5$ die oben angegebene Bedeutung besitzen,

mit einem Teil Amin der Formel III

$$H_2N\text{-R} \qquad (III)$$

in welcher

$R^1$ die oben angegebene Bedeutung besitzt,

oder dessen Salz mit einem Teil Aldehyd der Formel IX

$$X\text{-CHO} \qquad (IX)$$

in welcher

X die oben angegebene Bedeutung besitzt,

gegebenenfalls in Gegenwart von inerten organischen Lösungsmitteln bei Temperaturen zwischen 20 und 150°C umsetzt.

Überraschenderweise besitzen die erfindungsgemäßen 1-N-Aryl-dihydropyridin-ester der Formel I eine sehr starke kreislaufbeeinflussende insbesondere Coronarwirkung. Außerdem besitzen sie reaktionsfähige Gruppen und eignen sich daher zur Darstellung weiterer, pharmakologisch interessanter Präparate. Die erfindungsgemäßen Stoffe stellen somit eine Bereicherung der Pharmazie dar.

a) Verwendet man 3,4-Dimethoxybenzylidenacetessigsäuremethylester, Acetessigsäuremethylester (II) und p-Äthoxyanilin (III) (bzw. ß-(4-Äthoxyphenylamino)-crotonsäuremethylester (IV)) als Ausgangsstoffe, so kann der Reaktionsablauf für Variante a) durch folgendes Formelschema wiedergegeben werden:

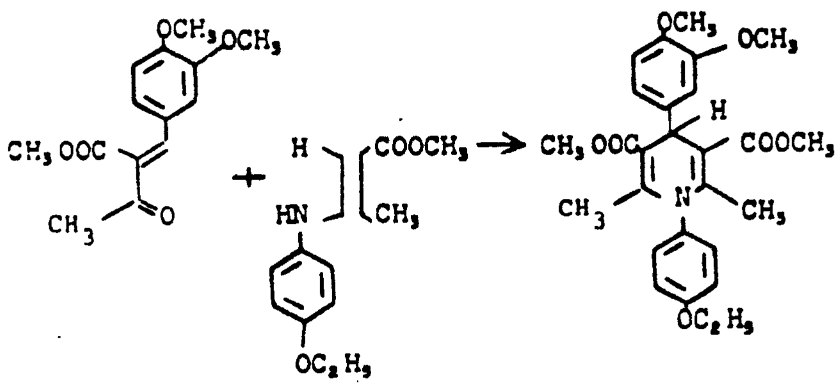

V IV (II+III) I

b) Verwendet man 3-Chlorbenzylidenacetessigsäureäthylester, Ptopionylessigsäureäthylester und p-Anisidin (bzw. ß-(4-Methoxyphenylamino)-ß-äthylacrylsäureäthylester (VII)) als Ausgangskomponenten, so kann der Reaktionsablauf für Variante b) durch das folgende Formelschema wiedergegeben werden:

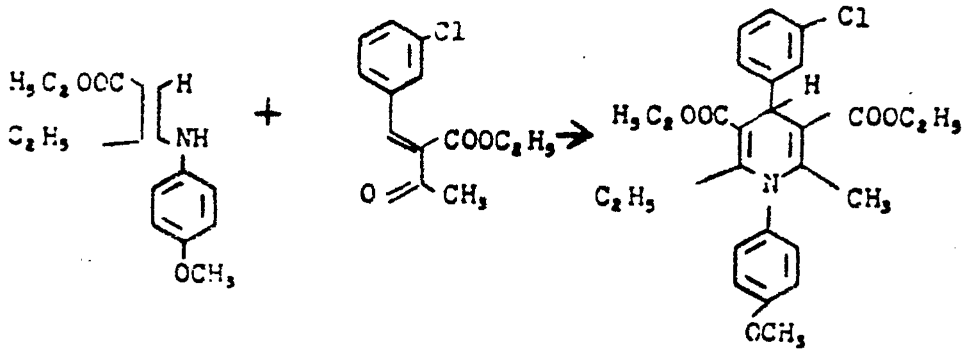

c) Verwendet man 3-Trifluormethylbenzaldehyd, ß-(4-Carbopropoxyphenylamino)-crotonsäurepropylester und γ-Propoxyacetessigsäureethylester als Ausgangskomponenten, so kann der Reaktionsablauf für Variante c) durch das folgende Formelschema wiedergegeben werden:

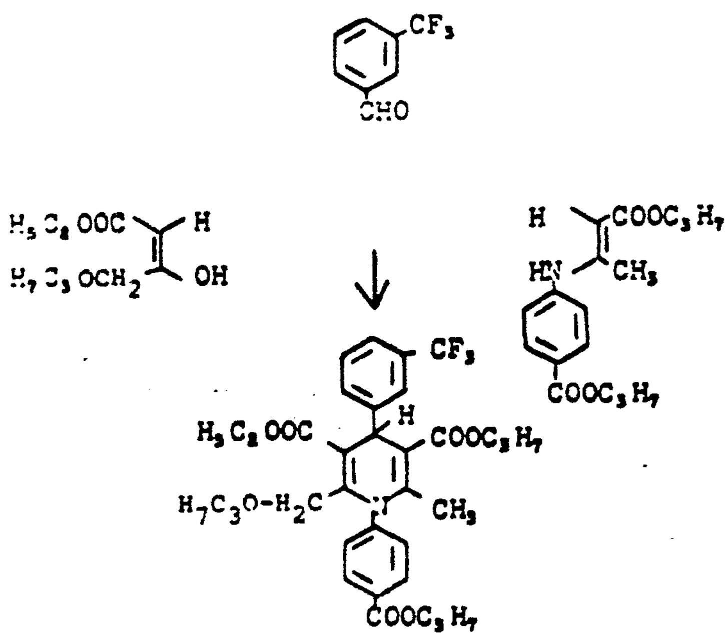

d) Verwendet man 3-Nitro-benzaldehyd, 2 Teile Acetessigsäureäthylester und p-Aminophenol, so kann der Reaktionsablauf für Variante d) durch das folgende Formelschema wiedergegeben werden :

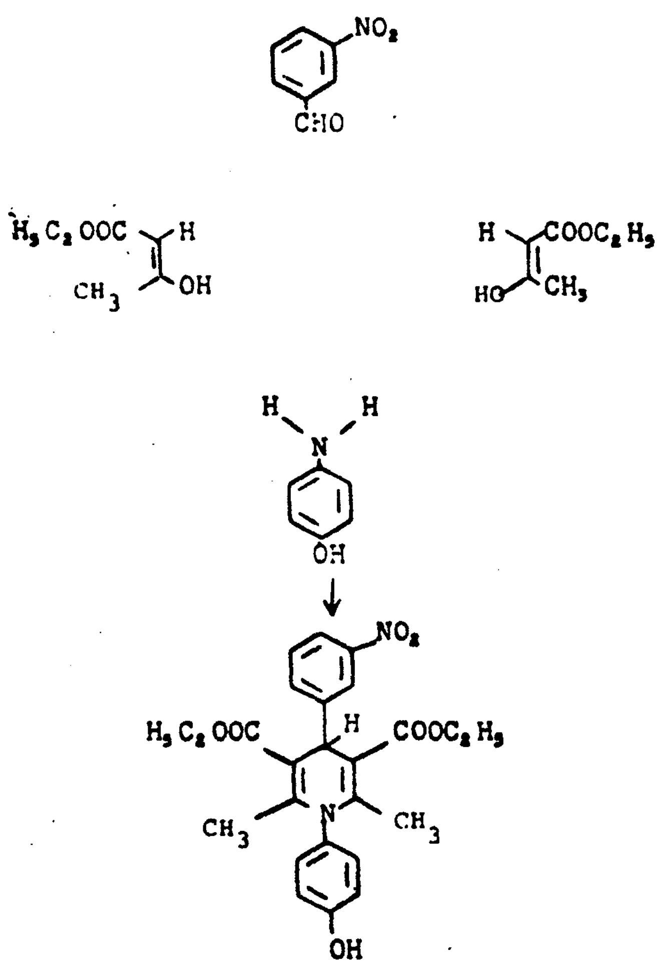

In der allgemeinen Formel I und in den Formeln II bis IX haben die Substituenten $R^1$, $R^2$, $R^3$, $R^4$, $R^5$ und X vorzugsweise die folgende Bedeutung:

$R^1$ steht vorzugsweise für einen Phenylrest der durch 1, 2 oder 3 gleiche oder verschiedene Substituenten aus der Gruppe Halogen, insbesondere Fluor, Chlor oder Brom, Hydroxy, Carboxy, Trifluormethyl, Nitro, Cyano, Alkyl, Alkoxy, Alkylmercapto, Alkylaminoalkyl und Dialkylaminoalkyl substituiert ist, wobei die vorgenannten Alkyl- und Alkoxy-Gruppen vorzugsweise je 1 bis 4 Kohlenstoffatome enthalten und gegegebenenfalls noch durch ein Sauerstoffatom in der Kette oder in der Anknüpfungsstelle zum Phenylring unterbrochen sind,

$R^2$ und $R^5$ die gleich oder verschieden sein können, stehen vorzugsweise für Wasserstoff oder Alkyl mit 1 bis 4 Kohlenstoffatomen, wobei die Alkylkette gegebenenfalls durch ein Sauerstoffatom unterbrochen ist,

$R^3$ und $R^4$ die gleich oder verschieden sein können, stehen vorzugsweise für Alkyl, Alkenyl oder Alkinyl mit bis zu 6, insbesondere mit bis zu 4 Kohlenstoffatomen, wobei die Alkyl- und Alkenylketten gegebenenfalls durch ein Sauerstoff- oder Stickstoffatom in der Kette unterbrochen sind und

X steht vorzugsweise für einen Alkylrest mit 1 bis 4 Kohlenstoffatomen, für einen Phenylrest, der gegebenenfalls durch 1 oder 2 Substituenten aus der Gruppe Nitro, Halogen, insbesondere Fluor, Chlor oder Brom, Trifluormethyl, Amino, Alkylamino, Dialkylamino, Alkyl, Alkoxy, Alkoxyalkyl, Alkylmercapto, Alkenyl und Alkinyl substituiert ist, wobei die genannten Alkyl-, Alkoxy-, Alkenyl- und Alkinyl-Reste jeweils bis zu 4 Kohlenstoffatome enthalten
oder
vorzugsweise für einen gegebenenfalls durch Halogen, Nitro, Trifluormethyl, Alkoxy oder Alkyl mit je 1 oder 2 Kohlenstoffatomen substituierten Pyridylrest, Thienylrest, Naphthylrest oder Chinolylrest.

Falls nicht ausdrücklich anders angegeben bedeutet der Ausdruck Alkyl in der vorliegenden Anmeldung geradkettiges, verzweigtes oder cyclisches Alkyl mit 1 bis 8 Kohlenstoffatomen, insbesondere mit 1 bis 6 Kohlenstoffatomen. Beispielhaft seien genannt Methyl, Äthyl, Propyl, Isopropyl, n-Butyl, iso-Butyl, tert.-Butyl, n-Pentyl, iso-Pentyl, n-Hexyl, Cyclohexyl, Cycloheptyl, n-Heptyl, n-Octyl, iso-Octyl, Cyclopropyl und Cyclobutyl.

Der Ausdruck Alkenyl steht vorzugsweise für geradkettiges, verzweigtes oder cyclisches Alkenyl mit 2 bis 6, insbesondere 2 bis 4, Kohlenstoffatomen. Beispielhaft seien genannt Propenyl-(2), Butenyl-(3), Pentenyl-(2) und Cyclohexenyl.

Der Ausdruck Alkinyl steht vorzugsweise für geradkettiges oder verzweigtes Alkinyl mit bis zu 6 Kohlenstoffatomen. Beispielhaft seien genannt Propinyl-(2) und Butinyl-(3).

Die vorgenannten Alkyl-, Alkenyl- und Alkinylreste sind gegebenenfalls durch Sauerstoffatome oder Stickstoffatome in der Kette unterbrochen, insbesondere durch ein Sauerstoffatom.

Halogen steht vorzugsweise für Fluor, Chlor, Brom oder Jod, insbesondere für Fluor oder Chlor.

Der Arylrest des Substituenten X kann vorzugsweise 1 bis 3, insbesondere 1 oder 2 gleiche oder verschiedene Substituenten tragen. Als Substituenten seien aufgeführt: Phenyl, Alkyl mit vorzugsweise 1 bis 4, insbesondere 1 oder 2 Kohlenstoffatomen, wie Methyl, Aethyl, n- und i-Propyl und n-, i- und t-Butyl; Alkoxy mit vorzugsweise 1 bis 4, insbesondere 1 oder 2 Kohlenstoffatomen, wie Methoxy, Aethoxy, n- und i-Propyl- und n-, i- und t-Butyloxy; Trifluormethyl; Trifluormethoxy; Hydroxy;
Halogen, vorzugsweise Fluor, Chlor, Brom und Jod, insbesondere Chlor und Brom; Cyano; Nitro; Azido; Amino; Monoalkyl- und Dialkylamino mit vorzugsweise 1 bis 4, insbesondere 1 oder 2 Kohlenstoffatomen je Alkylgruppe, wie Methylamino, Methyläthylamino, n- und i-Propylamino und Methyl-n-butylamino; Carbalkoxy mit vorzugsweise 2 bis 4, insbesondere 2 oder 3 Kohlenstoffatomen, wie Carbomethoxy und Carboäthoxy; Acylamino mit vorzugswiese 1 bis 4, insbesondere 2 oder 3 Kohlenstoffatomen, wie Acetylamino und Propionylamino; Acyloxy mit vorzugsweise 2 bis 6, insbesondere 2 bis 4 Kohlenstoffatomen wie Acetyloxy und Propionyloxy; $S(O)_m$-Alkyl, worin m eine Zahl von 0 bis 2, insbesondere 0 oder 2 bedeutet und Alkyl vorzugsweise 1 bis 4, insbesondere 1 oder 2 Kohlenstoffatome enthält, wie Methylthio, Aethylthio, Methylsulfoxyl, Aethylsulfoxyl, Methylsulfonyl und Aethylsulfonyl.

Cycloalkyl bedeutet vorzugsweise mono-, bi- und tricyclisches Cycloalkyl mit vorzugsweise 3 bis 10, insbesondere 3, 5 oder 6 Kohlenstoffatomen. Beispielhaft seien genannt : Cyclopropyl, Cyclobutyl, Cyclopentyl, Cyclohexyl, Cycloheptyl, Bicyclo-/2,2,1/-heptyl, Bicyclo-/2,2,2/-octyl und Adamantyl.

Cycloalkenyl bedeutet vorzugsweise mono-, bi- und tricyclisches Cycloalkenyl mit vorzugsweise 5 bis 10, insbesondere 5, 6 oder 7 Kohlenstoffatomen. Beispielhaft seien Cyclopentenyl, Cyclohexenyl und Cycloheptenyl genannt.

Alkyl und Alkoxy als Substituenten im Naphthyl-, Chinolyl-, Isochinolyl-, Pyridyl-, Pyrimidyl-, Diphenyl-, Furyl- oder Pyrrylrest X bedeuten geradkettiges oder verzweigtes Alkyl und Alkoxy mit vorzugsweise 1 bis 6, insbesondere 1 bis 4 Kohlenstoffatomen. Beispielhaft seien Methyl, Aethyl, n- und i-Propyl, n-, i- und t-Butyl sowie Methoxy, Aethoxy, n- und i-Propoxy und n-, i- und t-Butoxy genannt.

Salze der Verbindungen der Formel I sind alle nichttoxischen, physiologisch verträglichen Säureadditionssalze. Als anorganische und organische Säuren, die mit den Verbindungen der Formel I solche Salze bilden, seien beispielhaft genannt : Halogenwasserstoffsäuren, z.B. Chlor- und Bromwasserstoffsäure, insbesonders Chlorwasserstoffsäure, Phosphorsäuren, Schwefelsäuren, Salpetersäure, mono- und bifunktionelle Carbonsäuren und Hydroxycarbonsäuren, z.B.Essigsäure, Maleinsäure, Bernsteinsäure, Fumarsäure, Weinsäure, Zitronensäure, Salicylsäure, Sorbinsäure, Milchsäure und 1,5-Naphthalincarbonsäure.

Die Salze werden nach allgemein üblichen Methoden, z.B. durch Auflösen der Base in Aether und Versetzen der Lösung mit der betreffenden Säure hergestellt.

Die erfindungsgemäß verwendbaren β-Ketocarbonsäureester der Formel II und VI sind bekannt oder können nach bekannten Verfahren hergestellt werden (vgl. B. Johnson und H. Chesnoff, J.A.C.S. 36, 1744 (1914) ).

Als Beispiele seien genannt:

β-Ketocarbonsäureester

Formylessigsäureäthylester, Acetessigsäuremethylester, Acetessigsäureäthylester, Acetessigsäurepropylester, Acetessigsäureisopropylester, Acetessigsäurebutylester, Acetessigsäure-t-butylester, Acetessigsäure-(α- oder β-)-methoxyäthylester, Acetessigsäure-(α- oder β-)-äthoxyäthylester, Acetessigsäure-(α- oder β-)-propoxyäthylester, Acetessigsäure-(α- oder β-)-hydroxyäthylester, Acetessigsäureallylester, Acetessigsäurepropargylester, Acetessigsäurecyclohexylester, Propionylessigsäuremethylester, Propionylessigsäureäthylester, Propionylessigsäureisopropylester, Butyrylessigsäureäthylester, Acetessigsäureallylester, Acetessigsäurepropargylester, Acetessigsäure-(α- oder γ-hydroxyallyl)ester, γ-Methoxyacetessigsäuremethylester, γ-Methoxyacetessigsäureäthylester, γ-Methoxyacetessigsäurepropylester, γ-Methoxyacetessigsäurebutylester, γ-Aethoxyacetessigsäureäthylester, γ-Propoxyacetessigsäureisopropylester, γ-Isopropoxyacetessigsäuremethylester, γ-Isobutoxyacetessigsäurepropylester, γ-Methoxypropionylessigsäureäthylester, γ-Propoxypropionylessigsäurepropylester.

Die erfindungsgemäß verwandten Aniline (III) sind bekannt oder können nach bekannten Verfahren hergestellt werden (z.B.: Conrad, Limpach, B 20, 944 (1887)).

Als Beispiele seien genannt:

2,3- oder 4-Methoxyanilin, 2,3- oder 4-Aethoxyanilin, 2,3- oder 4-Propoxyanilin, 2,3- oder 4-Isopropoxyanilin, 2,3- oder 4-Butoxyanilin, 2,3- oder 4-Methylanilin, 2,3- oder 4-Aethylanilin, 2,3- oder 4-Propylanilin, 2,3- oder 4-Isopropylanilin, 2,3- oder 4-(β-Methylaminoäthoxy)-anilin, 2,3- oder 4-( Dimethylaminoäthoxy)-anilin, 2,4- oder 4-(β-Diäthylaminoäthoxy)-anilin, 2,3- oder 4-(β-Dipropylaminobutoxy)-anilin, 2,3- oder 4-( -Dipropylaminopropoxy)-anilin, 2,3- oder 4-(β-Hydroxy- -tert.Butylaminopropoxy)-anilin, 2-Chlor-4-methoxyanilin, 3-Brom-4-Aethylmercaptoanilin, 3-Jod-4-propoxyanilin, 3-Isopropoxy-4-fluoranilin, 2,3- oder 4-Hydroxyanilin, 2-Hydroxy-4-chloranilin, 2,4-Dimethoxyanilin, 2,5-Diäthoxyanilin, 2,3- oder 4-Carboxyanilin, 2,3- oder 4 Carbmethoxyanilin, 2,3- oder 4-Carbäthoxyanilin, 2,3- oder 4-Carbpropoxyanilin, 2-Methoxy-4-carboxyanilin, 2-Aethoxy-4-carbäthoxyanilin, 2-Hydroxy-4-carboxyanilin, 3-Hydroxy-4-carboxyanilin, 4-Hydroxy-3-carboxyanilin, 5-Hydroxy-4-carboxyanilin, 5-Propoxy-3-carboxyanilin, 2,4-Dioxyanilin, 2,5-Dioxyanilin, 2,3-Dioxyanilin, 2,4-Dioxy-5-carboxyanilin, 2,4-Dioxy-3-carbbutoxyanilin, 2,5-Dimethoxy-4-carbmethoxyanilin.

Die erfindungsgemäß verwandten β-Anilino-crotonsäureester der Formeln IV und VII sind bekannt oder können nach bekannten Verfahren hergestellt werden (z.B. Conrad, Limpach B 20, 944 (1887)).

β-Anilino-crotonsäureäthylester, β-(4-methoxyanilino)-crotonsäuremethylester, β-(3-Methoxyanilino)-crotonsäure-isopropylester, β-(2-Methoxyanilino)-crotonsäureäthylester, β-(3-Isopropoxyanilino)-crotonsäure-isopropylester, β-(3-Hydroxyanilino)-crotonsäurebutylester, β-(2-Methyl-anilino)-crotonsäurepropylester, β-(4-Caräthoxyanilino)-crotonsäureäthylester, β-(2-Methylmercaptoanilino)-crotonsäuremethylester, β-(3-Chlor-4-methoxyanilino)-crotonsäure-(β-äthoxy-äthyl)-ester, β-(2-Methoxy-5-bromanilino)-crotonsäurepropylester, β-(2-Toluidino)-crotonsäuremethylester, β-(3-Toluidino)-crotonsäureäthylester, β-(4-Toluidino)-crotonsäureallylester, β-(4-Methoxyanilino)-crotonsäurepropylester, β-(2-Aethoxyanilino)-crotonsäureisopropylester, β-(3-Propoxyanilino)-crotonsäurebutylester, β-(2-Chlor-4-methoxyanilino)-crotonsäurepropylester, β-Methyl-β-(3-methoxy-4-fluoranilino)-crotonsäure-(β-äthoxyäthyl)ester, β-(4-Hydroxyanilino)-crotonsäure-isobutylester, β-Aethyl-β-(2,4-dimethoxyanilino)-crotonsäureäthylester, β-(2-Butoxy-5-bromanilino)-crotonsäure-(β-hydroxyäthyl)-ester, β-(2-Methylmercaptoanilino)-crotonsäurepropargylester, β-Aethyl-β-(4-äthylmercaptoanilino)-crotonsäure-tert.-butylester.

Die erfindungsgemäß verwendbaren Yliden-β-ketocarbonsäureester der Formel VIII sind teilweise bekannt oder können nach allgemein bekannten Methoden hergestellt werden (Org. Reactions XI, 204 ff, (1967) ).

Als Beispiele seien genannt:

Yliden-β-ketocarbonsäureester:

Benzylidenacetessigsäuremethylester,
2'-Nitrobenzyliden-acetessigsäuremethylester,
3'-Nitrobenzylidenacetessigsäurepropargylester,
3'-Nitrobenzylidenacetessigsäureallylester,
3'-Nitrobenzylidenacetessigsäure-β-äthoxyäthylester,
4'-Nitrobenzylidenacetessigsäureisopropylester,
3'-Nitro-6'-chlorbenzylidenacetessigsäuremethylester,
2'-Cyanbenzylidenpropionylessigsäureäthylester,
3'-Cyanbenzylidenacetessigsäuremethylester,
3'-Nitro-4'-chlorbenzylidenacetessigsäure-tert.-butylester,
2'-Nitro-4'-methoxybenzylidenacetessigsäuremethylester,
2'-Cyan-4'-methylbenzylidenacetessigsäureäthylester,
2'-Azidobenzylidenacetessigsäureäthylester,
2'-Methylmercaptobenzylidenacetessigsäureisopropylester,
2'-Sulfinylmethylbenzylidenacetessigsäureäthylester,
2-Sulfonylmethylacetessigsäureäthylester,
(2'-Aethoxy-1'-naphthyliden)-Acetessigsäuremethylester,
α-Pyridylmethylidenacetessigsäuremethylester,
α-Pyridylmethylidenacetessigsäureallylester,
α-Pyridylmethylidenacetessigsäurecyclohexylester,
(6-Methyl-α-pyridyl)-methylidenacetessigsäureäthylester,
4',6'-Dimethoxy-(5'-pyrimidyl)-methylidenacetessigsäureäthylester
(2'-Thenyl)-methylidenacetessigsäureäthylester,
(2'-Furyl)-methylidenacetessigsäureallylester,

(2'-Pyrryl)-methylidenacetessigsäuremethylester,
α-Pyridylmethylidenpropionylessigsäuremethylester,
2'-, 3'- oder 4'-Methoxybenzylidenacetessigsäureäthylester,
2'-Isopropoxybenzylidenacetessigsäureäthylester,
3'-Butoxybenzylidenacetessigsäuremethylester,
3',4',5'-Trimethoxybenzylidenacetessigsäureallylester,
2'-Methylbenzylidenacetessigsäure-β-propoxyäthylester,
3',4'-Dimethoxy-5'-brombenzylidenacetessigsäureäthylester,
2'-, 3'- oder 4'-Chlor/Brom/Fluor/-Jodbenzylidenacetessigsäureäthylester,
3'-Chlorbenzylidenpropionylessigsäureäthylester,
2'-, 3'- oder 4'-Trifluormethylbenzylidenacetessigsäurepropylester,
2'-Carbäthoxybenzylidenacetessigsäureäthylester,
4'-Carboxyisopropylbenzylidenacetessigsäureisopropylester,
4'-Sulfonylmethylbenzylidenacetessigsäureäthylester,
(1'-Naphthyliden)acetessigsäureäthylester,
(2'-Naphthyliden)acetessigsäureäthylester,
(2-Methoxy-1'-naphthyliden)acetessigsäureäthylester,
(5'-Brom-1'-naphthyliden)acetessigsäuremethylester,
(2'-Chinolyl)-methylidenacetessigsäureäthylester,
(4'-Chinolyl)-methylidenacetessigsäureäthylester,
(8'-Chinolyl)methylidenacetessigsäureäthylester,
(1'-Isochinolyl)methylidenacetessigsäuremethylester,
α-Pyridylmethylidenacetessigsäuremethylester,
α-Pyridylmethylidenacetessigsäureallylester,
α-Pyridylmethylidenacetessigsäurepropargylester,
α-Pyridylmethylidenacetessigsäurecyclohexylester,
β-Pyridylmethylidenacetessigsäure-β-(äthoxyäthyl)ester,
β-Pyridylmethylidenacetessigsäureäthylester,
γ-Pyridylmethylidenacetessigsäuremethylester

(2'-Pyrryl)-acetessigsäuremethylester,
(3'-Nitro-benzyliden)-propionylessigsäureäthylester,
α-Pyridylmethylidenpropionylessigsäuremethylester,
Benzyliden-γ-methoxyacetessigsäureäthylester,
2'-Nitrobenzyliden-γ-methoxyacetessigsäureäthylester,
3'-4'-Dimethoxybenzyliden-γ-propoxyacetessigsäurepropylester,
2'-Trifluormethylbenzyliden-γ-methoxyacetessigsäureäthylester,
2-Chlorbenzyliden-γ-äthoxyacetessigsäurepropylester.

Die erfindungsgemäß verwendbaren Aldehyde der Formel IX sind bereits bekannt oder können nach bekannten Methoden hergestellt werden (E. Mosettig, Org. Reactions, VIII, 218 ff, (1954)).

Als Beispiele seien genannt:
Aldehyde:
Benzaldehyd, 2-, 3- oder 4-Methoxybenzaldehyd, 2-Isopropoxybenzaldehyd, 3-Butoxybenzaldehyd, 3,4-Dioxymethylenbenzaldehyd, 3,4,5-Trimethoxybenzaldehyd, 2-, 3- oder 4-Chlor/Brom/Jod/Fluorbenzaldehyd, 2,4- oder 2,6-Dichlorbenzaldehyd, 2,4-Dimethylbenzaldehyd, 3,5-Diisopropyl-4-methoxybenzaldehyd, 2-, 3- oder 4-Nitrobenzaldehyd, 2,4- oder 2,6-Dinitrobenzaldehyd, 2-Nitro-6-brombenzaldehyd, 2-Nitro-3-methoxy-6-chlorbenzaldehyd, 2-Nitro-4-chlorbenzaldehyd, 2-Nitro-4-methoxybenzaldehyd, 2-, 3- oder 4-Trifluormethylbenzaldehyd, 2-, 3- oder 4-Dimethylaminobenzaldehyd, 4-Dibutylaminobenzaldehyd, 4-Acetaminobenzaldehyd, 2-, 3- oder 4-Cyanbenzaldehyd, 2-Nitro-4-cyanbenzaldehyd, 3-Chlor-4-cyan-benzaldehyd, 2-, 3- oder 4-Methylmercaptobenzaldehyd, 2-Methylmercapto-5-nitrobenzaldehyd, 2-Butylmercaptobenzaldehyd, 2-, 3- oder 4-Methylsulfinylbenzaldehyd, 2-, 3- oder 4-Methylsulfonylbenzaldehyd, Benzaldehyd-2-carbonsäureäthylester, Benzaldehyd-3-carbonsäureisopropylester, Benzaldehyd-4-carbonsäurebutylester, 3-Nitrobenzaldehyd-4-carbonsäureäthylester, Zimtaldehyd, Hydrozimtaldehyd, Formylcyclohexan, 1-Formylcyclohexan-3, 1-Formyl-cyclohexin-1,3, 1-Formyl-cyclopenten-3, α-, ß- oder γ-Pyridinaldehyd, 6-Methylpyridin-2-aldehyd, Furan-2-aldehyd, Thiophen-2-aldehyd und Pyrrol-2-aldehyd, N-Methylpyrrol-2-aldehyd, N-Methylpyrrol-2-aldehyd, 2-, 3- oder 4-Azidobenzaldehyd, Pyrimidin-4-aldehyd, 5-Nitro-6-methylpyridin-2-aldehyd, 1- oder 2-Naphthaldehyd, 5-Brom-1-naphthaldehyd, Chinolin-2-aldehyd, 7-Methoxy-chinolin-4-aldehyd, Isochinolin-1-aldehyd, 3- oder 4-Hydroxybenzaldehyd.

Als Verdünnungsmittel kommen bei den Verfahrensvarianten a) bis e) Wasser und alle inerten organischen Lösungsmittel in Frage. Hierzu gehören vorzugsweise Alkohole, z.B. niedere Alkylalkohole mit vorzugsweise 1 bis 4 Kohlenstoffatomen, wie Äthanol, Methanol, Isopropanol, Äther, z.B. niedere Dialkyläther (vorzugsweise 3 bis 5 Kohlenstoffatome), wie Diäthyläther oder Ringäther wie Tetrahydrofuran, Dioxan, niedere aliphatische Carbonsäuren (vorzugsweise 2 bis 5 Kohlenstoffatome), wie Essigsäure, Propionsäure, niedere Dialkylformamide (vorzugsweise 1 oder 2 Kohlenstoffatome je Alkylgruppe), wie Dimethylformamid, niedere Alkylnitrile (vorzugsweise 2 bis 4 Kohlenstoffatome), wie Acetonitril, Dimethylsulfoxid, flüssige heteroaromatische Basen wie Pyridin, sowie Gemische dieser Lösungsmittel einschließlich Wasser untereinander.

Die Reaktionstemperaturen können bei den Verfahrensvarianten a) bis e) in einem größeren Bereich variiert werden. Im allgemeinen arbeitet man zwischen etwa 20 und etwa 150°C, vorzugsweise zwischen 50 und 100°C, insbesondere bei der Siedetemperatur des verwendeten Lösungsmittels.

Die Umsetzung kann bei Normaldruck, aber auch bei erhöhtem Druck durchgeführt werden. Im allgemeinen arbeitet man bei Normaldruck.

Bei der Durchführung der erfindungsgemäßen Verfahrensvarianten a) bis e) werden die an der Reaktion beteiligten Ausgangsstoffe vorzugsweise jeweils etwa in molaren Mengen eingesetzt. Das verwendete Amin bzw. dessen Salz wird zweckmäßig im Überschuß von 1 bis 2 Mol zugegeben. Die Molverhältnisse können über einen weiten Bereich variiert werden ohne, daß das Ergebnis nachteilig beeinflußt wird.

Als Wirkstoffe seien zusätzlich zu den in den Beispielen beschriebenen Verbindungen im einzelnen genannt:

N-(Isopropoxyphenyl)-2-äthyl-6-propyl-4-(3-Nitrophenyl)-1,4-dihydropyridin-3-carbonsäureäthylester-5-carbonsäurepropylester

N-(3-Butoxy-4-chlorphenyl)-2,6-dipropyl-4-(β-pyridyl)-1,4-dihydropyridin-3,5-dicarbonsäurediallylester

N-(2-Carbpropoxyisopropoxyphenyl-2,6-dibutoyl-4-(2-Nitrophenyl)-1,4-dihydropyridin-3-carbonsäurebutylester-5-carbonsäurepropargylester

N- 3-(γ-Isopropylamino-β-hydroxypropoxyphenyl)-2,6-dimethoxymethyl-4-(3,4,5-trimethoxyphenyl)-1,4-dihydropyridin-3,5-dicarbonsäure-dipropylester.

Die neuen Verbindungen sind als Arzneimittel, insbesondere als gefäß- und kreislaufbeeinflussende Wirkstoffe verwendbar.

Die erfindungsgemäßen Verbindungen haben ein breites und vielseitiges pharmakologisches Wirkungsspektrum.

Im einzelnen weisen die erfindungsgemäßen Verbindungen folgende Hauptwirkungen auf:

1) Die neuen Verbindungen bewirken bei parenteraler, oraler und perlingualer Gabe eine deutliche und langanhaltende Erweiterung der Coronargefäße.

   Diese Wirkung auf die Coronargefäße wird durch einen gleichzeitigen Nitrit-ähnlichen herzentlastenden Effekt verstärkt. Sie beeinflussen bzw. verändern den Herstoffwechsel im Sinne einer Energieersparnis.

2) Die neuen Verbindungen senken den Blutdruck von normotonen und hypertonen Tieren und können somit als antihypertensive Mittel verwendet werden.

3) Die Erregbarkeit des Reizbildungs- und Erregungsleitungssystems innerhalb des Herzens wird herabgesetzt, so daß eine in therapeutischen Dosen nachweisbare Antiflimmerwirkung resultiert.

4) Der Tonus der glatten Muskulatur der Gefäße wird unter der Wirkung der Verbindungen stark vermindert. Diese

gefäßspasmolytische Wirkung kann im gesamten Gefäßsystem stattfinden oder sich mehr oder weniger isoliert in umschriebenen Gefäßgebieten (wie z.B. dem Zentralnervensystem) manifestieren.

5) Die Verbindungen haben stark muskulär-spasmolytische Wirkungen, die an der glatten Muskulatur des Magens, Darmtraktes, des Urogenitaltraktes und des Respirationssystems deutlich werden.

6) Die Verbindungen beeinflussen den Cholesterin- bzw. Lipidspiegel des Blutes.

7) Die Verbindungen senken die Herzfrequenz.

Die neuen Verbindungen sind demnach zur Vorbeugung, Besserung oder Heilung von Erkrankungen geeignet, bei denen insbesondere die oben angegebenen Effekte erwünscht sind.

Zur vorliegenden Erfindung gehören pharmazeutische Zubereitungen, die neben nichttoxischen, inerten pharmazeutisch geeigneten Trägerstoffen eine oder mehrere Verbindungen der Formel I und/oder deren Salze enthalten oder die aus einer oder mehreren Verbindungen der Formel I und/oder deren Salzen bestehen sowie Verfahren zur Herstellung dieser Zubereitungen.

Zur vorliegenden Erfindung gehören auch pharmazeutische Zubereitungen in Dosierungseinheiten, Dies bedeutet, daß die Zubereitungen in Form einzelner Teile z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzukker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin, und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin, und Bentonit, und (i) Gleitmittel, z.B. Talkum, Calcium- und Magnesiumstearat und feste Polyäthylenglykole oder Gemische der unter (a) - (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffen auch in mirkoverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Lösungen und Emulsionen können neben dem oder den Wirkstoffen die üblichen Trägerstoffe wie Lösungsmittel, Lösungsvermittler und Emulgatoren, z.B. Wasser, Äthylalkohol, Isopropylalkohol, Äthylcarbonat, Äthylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Äthylalkohol, Propylenglykol, Suspendiermittel, z.B. äthoxylierte Isostearylalkohole, Polyoxyäthylensorbit- und -sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gewichtsprozent der Gesamtmischung vorhanden sein.

Die oben aufgeführten pharmazeutischen Zubereitungen können außer Verbindungen der Formel I und/oder deren Salzen auch andere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der Verbindungen der Formel I und/oder deren Salzen sowie von pharmazeutischen Zubereitungen, die eine oder mehrere Verbindungen der Formel I und/oder deren Salze enthalten, in der Human- und Veterinärmedizin zur Verhütung, Besserung und/oder Heilung der oben angeführten Erkrankungen.

Die Wirkstoffe oder die pharmazeutischen Zubereitungen können vorzugsweise oral, parenteral und/oder rectal, vorzugsweise oral und parenteral, insbesondere perlingual und intravenös appliziert werden.

Im allgemeinen hat es sich als vorteilhaft erwiesen, den oder die Wirkstoffe bei parenteraler (intravenöser) Applikation in Mengen von etwa 0,005 bis etwa 10, vorzugsweise von 0,02 bis 5 mg/kg Körpergewicht je 24 Stunden und bei oraler Applikation in Mengen von etwa 0,1 bis etwa 50, vorzugsweise 1 bis 30 mg/kg Körpergewicht je 24 Stunden, gegebenenfalls in Form Einzelgaben zur Erzielung der gewünschten Ergebnisse zu verabreichen. Eine Einzelgabe enthält den oder die Wirkstoffe, vorzugsweise in Mengen von etwa 0,002 bis etwa 5, insbesondere 0,01 bis 1 mg/kg Körpergewicht. Es kann jedoch erforderlich sein, von den genannten Dosierungen abzuweichen, und zwar in Abhängigkeit von der Art und dem Körpergewicht des zu behandelnden Objektes, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Mengen Wirkstoff auszukommen, während

in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die Herstellung der neuen Verbindung soll anhand der vorliegenden Ausführungsbeispiele exemplarisch erläutert werden.

Beispiel 1

N-(4-methoxyphenyl)-2,6-dimethyl-4(-β-pyridyl)-1,4-dihydropyridin-3,5-dicarbonsäurediäthylester

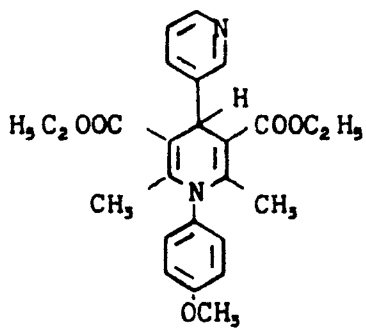

Man erhitzt 21,4 g Pyridin-3-aldehyd, 52 ccm Acetessigsäureäthylester und 24,6 g p-Anisidin in 50 ccm Aethanol über Nacht zum Sieden, kühlt und erhält in 60%iger Ausbeute hellgelbe Kristalle vom Fp. 148-150°C.

Auf gleiche Weise wurden dargestellt :

a) N-(4-methoxyphenyl)-2,6-dimethyl-4-(γ-pyridyl)-1,4-dihydropyridin-3,5-dicarbonsäurediäthylester
hellgraue Kristalle vom Fp. 120°C, Ausbeute: 50 %

b) N-(4-methoxyphenyl)-2,6-dimethyl-4-(α-pyridyl)-1,4-dihydropyridin-3,5-dicarbonsäurediäthylester
hellbraune Kristalle vom Fp. 134°C, Ausbeute: 55 %.

c) N-(4'-methoxyphenyl)-2,6-dimethyl-4-(β-pyridyl)-1,4-dihydropyridin-3,5-dicarbonsäurediallylester
weisse Kristalle vom Fp. 145°C, Ausbeute : 45%.

d) N-(4'-methoxyphenyl)-2,6-dimethyl-4-(3'-nitrophenyl)-1,4-dihydropyridin-3,5-dicarbonsäuredi-(β-methoxyäthylester) hellgelbe Kristalle vom Fp. 122°C, Ausbeute: 75 %.

Beispiel 2

N-(3'-hydroxyphenyl)-2,6-dimethyl-4-(γ-pyridyl)-1,4-dihydropyridin-3,5-dicarbonsäurediäthylester

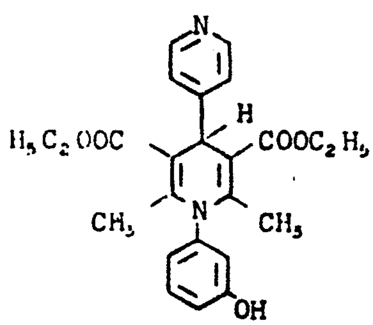

Man erhitzt 21 ccm Pyridin-4-aldehyd, 52 ccm Acetessigsäureäthylester und 21,8 g 3-Aminophenol in 80 ccm Aethanol über Nacht am Rückfluss zum Sieden und erhält in 50%iger Ausbeute Kristalle (beige) vom Fp. 166°C.

Auf gleiche Weise wurden erhalten

a) N-(2'-hydroxyphenyl)-2,6-dimethyl-4-(γ-pyridyl)-1,4-dihydropyridin-3,5-dicarbonsäurediäthylester Kristalle vom Fp. 197°C, Ausbeute : 35%.

Beispiel 3

N-(2'-4'-dimethoxyphenyl)-2,6-dimethyl-4-(β-pyridyl)-1,4-dihydropyridin-3,5-dicarbonsäurediäthylester

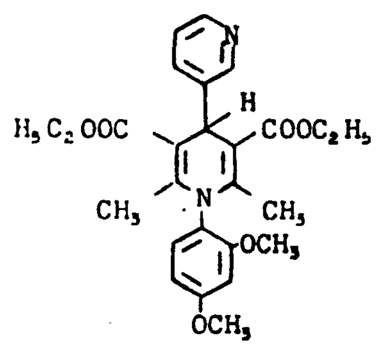

Man erhitzt 10 ccm Pyridin-3-aldehyd, 26 ccm Acetessigsäureäthylester und 15,3 g 4-Amino-1,3-dimethoxyanilin in 50 ccm Aethanol über Nacht zum Sieden und erhält nach dem Kühlen hellgraue Kristalle vom Fp. 119°C, Ausbeute : 55%.

Beispiel 4

A) N-(4'-Carbäthoxyisopropoxyphenyl)-2,6-dimethyl-4-(β-pyridyl)-1,4-dihydropyridin-3,5-dicarbonsäurediäthylester

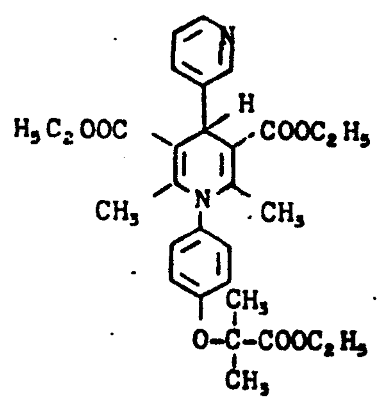

Man erhitzt die Lösung von 5,2 ccm Pyridin-3-aldehyd, 14 ccm Acetessigsäureäthylester und 11,2 g p-Carbäthoxyisopropoxyanilin in 40 ccm Alkohol 15-20 Stdn. zum Sieden, dampft i.V. ein und erhält aus Aether hellgraue Kristalle vom Fp. 106°C, Ausbeute : 25%.

B) N-(4'-carbäthoxyisopropoxyphenyl)-2,6-dimethyl-4-(3'-nitrophenyl)-1,4-dihydropyridin-3,5-dicarbonsäurediäthylester

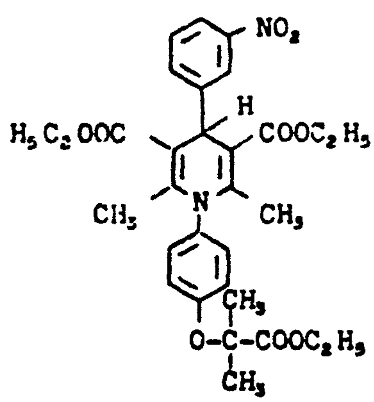

In die Lösung von 1,1 g Natrium in 50 ccm Aethanol trägt man 21,0 g N-(4'-hydroxyphenyl)-2,6-dimethyl-4-(3'-nitrophenyl)-1,4-dihydropyridin-3,5-dicarbonsäurediäthylester (Beispiel 8 ) und 9,5 g α-Bromisobuttersäureester ein, erhitzt über Nacht zum Sieden, gibt in Wasser und nimmt das Reaktionsprodukt in Aether auf. Aus Aethanol hellgelbe Kristalle vom Fp. 119-120°C, Ausbeute : 60%.

Beispiel 5

N-(3'-carboxyphenyl)-2,6-dimethyl-4-(γ-pyridyl)-1,4-dihydropyridin-3,5-dicarbonsäurediäthylester

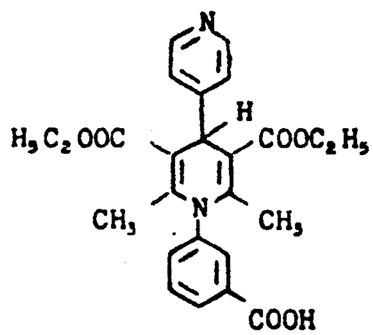

Man erhitzt 21 ccm Pyridin-4-aldehyd, 52 ccm Acetessigsäureäthylester und 27,4 g 3-Aminobenzoesäure in 150 ccm Aethanol über Nacht zum Sieden und erhält in 55%iger Ausbeute hellgelbe Kristalle vom Fp. 275°C.

Auf gleiche Weise werden erhalten

a) N-(3-carboxyphenyl)-2,6-dimethyl-4-(β-pyridyl)-1,4-dihydropyridin-3,5-dicarbonsäuredimethylester
hellbeige Kristalle vom Fp. 236°C, Ausbeute : 60%.

b) N-(3'-carboxyphenyl)-2,6-dimethyl-4-(β-pyridyl)-1,4-dihydropyridin-3,5-dicarbonsäurediäthylester
Kristalle (beige) vom Fp. 236-238°C, Ausbeute: 20 %.

c) N-(3'-carboxy-4'-chlorphenyl)-2,6-dimethyl-4-(β-pyridyl)-1,4-dihydropyridin-3,5-dicarbonsäurediäthylester
Kristalle (beige) vom Fp. 248-250°C, Ausbeute: 55 %.

d) N-(3'-carboxy)-2,6-dimethyl-4-(4'-chlorphenyl)-1,4-dihydropyridin-3,5-dicarbonsäurediäthylester
hellgraue Kristalle vom Fp. 206°C, Ausbeute: 25 %.

Beispiel 6

N-(2'-hydroxy-3'-carboxyphenyl)-2,6-dimethyl-4-(β-pyridyl)-1,4-dihydropyridin-3,5-dicarbonsäurediäthylester

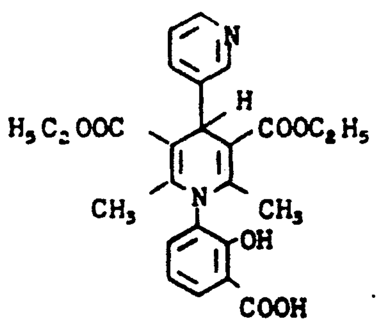

Die Lösung von 26 ccm Pyridin-3-aldehyd, 38,2 g 3-Amino-2-hydroxybenzoesäure und 65 ccm Acetessigsäureäthylester in 100 ccm Aethanol wird über Nacht zum Sieden erhitzt und anschliessend der Niederschlag heiss abgesaugt.
Kristalle (beige) vom Fp. 244°C, Ausbeute : 78%.

Auf gleiche Weise werden erhalten

a) N-(2'-hydroxy-3'-carboxyphenyl)-2,6-dimethyl-4-(γ-pyridyl)-1,4-dihydropyridin-3,5-dicarbonsäurediäthylester
goldgelbe Kristalle vom Fp. 236-238°C, Ausbeute: 80 %.

b) N-(2'-hydroxy-3'-carboxyphenyl)-2,6-dimethyl-4-(3'-nitrophenyl)-1,4-dihydropyridin-3,5-dicarbonsäurediäthylester goldgelbe Kristalle vom Fp. 236-238°C, Ausbeute: 80 %.

c) N-(3'-carboxy-4'-hydroxyphenyl)-2,6-dimethyl-4-(γ-pyridyl)-1,4-dihydropyridin-3,5-dicarbonsäurediäthylester Kristalle (oliv) vom Fp. 256°C, Ausbeute: 75 %.

d) N-(3'-carboxy-4'-hydroxy-6-methylphenyl)-2,6-dimethyl-4-(β-pyridyl)-1,4-dihydropyridin-3,5-dicarbonsäurediäthylester Kristalle (beige) vom Fp. 206°C, Ausbeute: 80 %.

e) N-(2'-hydroxy-3'-carboxyphenyl)-2,6-dimethyl-4-(γ-pyridyl)-1,4-dihydropyridin-3,5-dicarbonsäuredimethylester hellbraune Kristalle vom Fp. 245°C.

f) N-(3'-carboxy-4'-hydroxyphenyl)-2,6-dimethyl-4-(β-pyridyl)-1,4-dihydropyridin-3,5-dicarbonsäuredimethylester hellgraue Kristalle vom Fp. 245-247°C, Ausbeute: 90 %.

<u>Beispiel 7</u>

N-(4'-äthoxyphenyl)-2,6-dimethyl-4-(β-pyridyl)-1,4-dihydropyridin-3,5-dicarbonsäurediäthylester

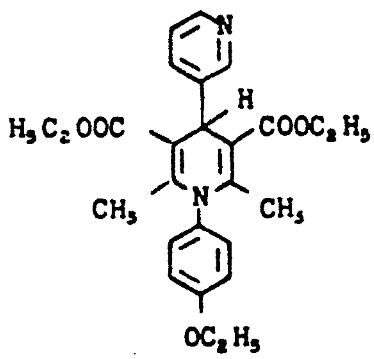

Die Lösung von 10 ccm Pyridin-3-aldehyd, 26 ccm Acetessigsäureäthylester und 13,7 g p-Phenetidin in 50 ccm Aethanol wird über Nacht zum Sieden erhitzt und anschliessend gekühlt. Weisse Kristalle vom Fp. 124°C, Ausbeute : 50%.

Auf gleiche Weise wurden erhalten

a) N-(4'-äthoxyphenyl)-2,6-dimethyl-4-(α-pyridyl)-1,4-dihydropyridin-3,5-dicarbonsäurediäthylester, Fp. 106°C, beige Kristalle, Ausbeute : 20%.

b) N-(4'-äthoxyphenyl)-2,6-dimethyl-4-(4'-nitrophenyl)-1,4-dihydropyridin-3,5-dicarbonsäurediäthylester Fp. 170°C, kanariengelbe Kristalle , Ausbeute: 70 %.

c) N-(4'-Aethoxyphenyl)-2,6-dimethyl-4-(2'-nitrophenyl)-1,4-dihydropyridin-3,5-dicarbonsäurediäthylester gelbe Kristalle vom Fp. 142°C, Ausbeute: 40 %.

d) N-(4'-Aethoxyphenyl)-2,6-dimethyl-4-(2'-trifluormethylphenyl)-1,4-dihydropyridin-3,5-dicarbonsäurediäthylester hellgelbe Kristalle vom Fp. 156-158°C, Ausbeute: 35 %.

e) N-(4'-Aethoxyphenyl)-2,6-dimethyl-4-(3'-trifluormethyl-phenyl)-1,4-dihydropyridin-3,5-dicarbonsäurediäthylester weiße Kristalle vom Fp. 138°C, Ausbeute: 60 %.

f) N-(4'-Aethoxyphenyl)-2,6-dimethyl-4-(3'-nitrophenyl)-1,4-dihydropyridin-3,5-dicarbonsäuredimethylester Kristalle hellbeige vom Fp. 136°C, Ausbeute: 80 %.

g) N-(4'-Aethoxyphenyl)-2,6-dimethyl-4-(3'-nitrophenyl)-1,4-dihydropyridin-3,5-dicarbonsäuredi-isopropylester hellgelbe Kristalle vom Fp. 168°C, Ausbeute: 55 %.

h) N-(4'-Aethoxyphenyl)-2,6-dimethyl-4-(3'-nitrophenyl)-1,4-dihydropyridin-3-carbonsäureäthylester-5-carbonsäure-methylester. Gelbe Kristalle vom Fp. 136°C, Ausbeute: 40 %.

i) N-(4'-Aethoxyphenyl)-2,6-dimethyl-4-(3'-nitrophenyl)-1,4-dihydropyridin-3,5-dicarbonsäurediäthylester grüngelbe Kristalle vom Fp. 128°C, Ausbeute: 65 %.

**Beispiel 8**

N-(4'-hydroxyphenyl)-2,6-dimethyl-4-(3'-nitrophenyl)-1,4-dihydropyridin-3,5-dicarbonsäurediäthylester

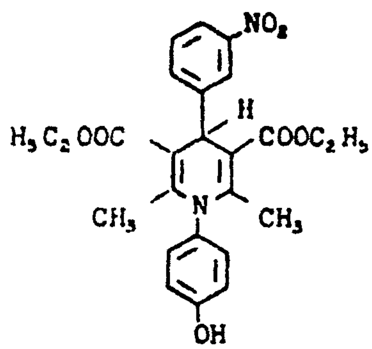

Nach 20 Stdn. Kochen einer Lösung von 75,5 g 3-Nitrobenzaldehyd, 55 g p-Aminophenol und 130 ccm Acetessigsäureäthylester in 200 ccm Aethanol und anschliessendem Kühlen erhält man in 65%iger Ausbeute gelbgrüne Kristalle vom Fp. 190°C.

Auf gleiche Weise wurden erhalten

a) N-(4'-hydroxyphenyl)-2,6-dimethyl-(β-pyrididyl)-1,4-dihydropyridin-3,5-dicarbonsäurediäthylester
Fp. 200°C, hellgelbe Kristalle, Ausbeute: 70 %.

b) N-(4-Hydroxyphenyl)-2,6-dimethyl-4-(4'-nitrophenyl)-1,4-dihydropyridin-3,5-dicarbonsäurediäthylester
hellgraue Kristalle vom Fp. 176°C, Ausbeute: 55 %.

c) N-(2-Hydroxyphenyl)-2,6-dimethyl-4-(γ-pyridyl)-1,4-dihydropyridin-3,5-dicarbonsäurediäthylester
beige Kristalle vom Fp. 198-200°C, Ausbeute : 35%.

d) N-(4-Hydroxyphenyl)-2,6-dimethyl-4-(4'-chlorphenyl)-1,4-dihydropyridin-3,5-dicarbonsäurediäthylester
hellgraue Kristalle vom Fp. 140-142°C, Ausbeute: 60 %

e) N-(4'-Hydroxyphenyl)-2,6-dimethyl-4-(γ-pyridyl)-1,4-dihydropyridin-3,5-dicarbonsäurediäthylester
hellbraune Kristalle vom Fp. 240°C, Ausbeute: 75 %.

f) N-(4'-Hydroxyphenyl)-2,6-dimethyl-4-(3'-nitro-6'-chlorphenyl)-1,4-dihydropyridin-3,5-dicarbonsäurediäthylester
gelbe Kristalle vom Fp. 320°C, Ausbeute: 35 %.

g) N-(4'-Hydroxyphenyl)-2,6-dimethyl-4-(2'-trifluormethylphenyl)-1,4-dihydropyridin-3,5-dicarbonsäurediäthylester hellbeige Kristalle vom Fp. 184°C, Ausbeute: 30 %

h) N-(4'-Hydroxyphenyl)-2,6-dimethyl-4-(3'-trifluormethylphenyl)-1,4-dihydropyridin-3,5-dicarbonsäurediäthylester
hellgraue Kristalle vom Fp. 124-126°C, Ausbeute: 35 %.

i) N-(2'-Hydroxy-5-chlorphenyl)-2,6-dimethyl-4-(β-pyridyl)-1,4-dihydropyridin-3,5-dicarbonsäurediäthylester hellbraune Kristalle vom Fp. 218-220°C, Ausbeute: 50 %.

j) N-(4'-Hydroxyphenyl)-2,6-dimethyl-4-(2'-nitrophenyl)-1,4-dihydropyridin-3,5-dicarbonsäurediäthylester hellgelbe Kristalle vom Fp. 204-206°C, Ausbeute: 45 %.

k) N-(4'-Hydroxyphenyl)-2,6-dimethyl-4-(3'-trifluormethyl-phenyl)-1,4-dihydropyridin-3,5-dicarbonsäurediäthylester hellgraue Kristalle vom Fp. 195°C.

l) N-(4'-Hydroxyphenyl)-2,6-dimethyl-4-(3'-trifluormethyl-phenyl)-1,4-dihydropyridin-3,5-dicarbonsäurediiso-propylester hellbraune Kristalle vom Fp. 165°C, Ausbeute: 35 %.

m) N-(4'-Hydroxyphenyl)-2,6-dimethyl-4-(3'-4'-5'-trimethoxyphenyl)-1,4-dihydropyridin-3,5-dicarbonsäurediäthylester hellbraune Kristalle vom Fp. 170-172°C, Ausbeute: 45 %.

n) N-(4'-Hydroxyphenyl)-2,6-dimethyl-4-(2'-4'-dichlorphenyl)-1,4-dihydropyridin-3,5-dicarbonsäurediäthylester Kristalle (beige) vom Fp. 196°C, Ausbeute: 25 %.

Beispiel 9

N-(4'-carboxyphenyl)-2,6-dimethyl-4-(4'-chlorphenyl)-1,4-dihydropyridin-3,5-dicarbonsäurediäthylester

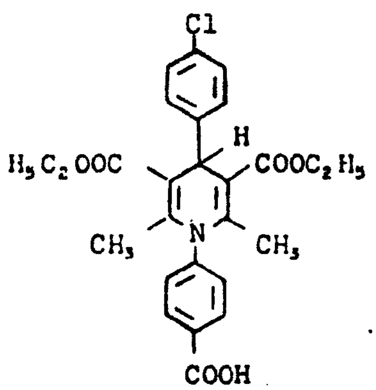

Man erhitzt 28,1 g 4-Chlorbentaldehyd, 52 ccm Acetessigsäureäthylester und 27,4 g p-Aminobenzoesäure in 180 ccm Aethanol über Nacht zum Sieden, kühlt und erhält in 30%iger Ausbeute hellgelbe Kristalle vom Fp. 215°C.

Auf gleiche Art werden erhalten

a) N-(4'-carboxyphenyl)-2,6-dimethyl-4-(3'-nitrophenyl)-1,4-dihydropyridin-3,5-dicarbonsäurediäthylester hellgelbe Kristalle vom Fp. 248°C, Ausbeute: 50 %.

b) N-(4'-carboxyphenyl)-2,6-dimethyl-4-(4'-nitrophenyl)-1,4-dihydropyridin-3,5-dicarbonsäurediäthylester hellgelbe Kristalle vom Fp. 216°C, Ausbeute: 40 %.

c) N-(4'-carboxyphenyl)-2,6-dimethyl-4-(β-pyridyl)-1,4-dihydropyridin-3,5-dicarbonsäurediäthylester Kristalle (hellbeige) vom Fp. 245°C, Ausbeute: 40 %.

Beispiel 10

N-(4'-methoxyphenyl)-2,6-dimethyl-4-(3'-trifluormethyl-phenyl)-1,4-dihydropyridin-3,5-dicarbonsäurediäthylester

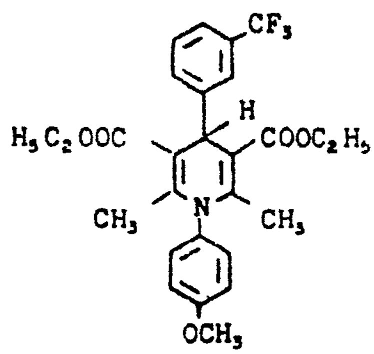

Man erhitzt 17,4 g m-Trifluormethylbenzaldehyd, 42 ccm Acetessigsäureäthylester und 12,3 g p-Anisidin in 40 ccm Aethanol zum Sieden über Nacht, kühlt und erhält hellgelbe Kristalle vom Fp. 121-122°C, Ausbeute : 45%.

a) Auf gleiche Weise wird mit 17,4 g o-Trifluormethylbenzaldehyd der N-(4-methoxyphenyl)-2,6-dimethyl-4-(2'-trifluormethyl-phenyl)-1,4-dihydropyridin-3,5-dicarbonsäurediäthylester vom Fp. 164-166°C in hellgelben Kristallen und 30%iger Ausbeute erhalten.

b) N-(4'-methoxyphenyl)-2,6-dimethyl-4-(4'-nitrophenyl)-1,4-dihydropyridin-3,5-dicarbonsäurediäthylester Fp. 138-140°C, gelbe Kristalle, Ausbeute: 50 %.

c) N-(4'-methoxyphenyl)-2,6-dimethyl-4-(3'-nitrophenyl)-1,4-dihydropyridin-3,5-dicarbonsäurediäthylester hellgelbe Kristalle vom Fp. 130°C, Ausbeute: 75 %.

d) N-(4-methoxyphenyl-2,6-dimethyl-4-(3'-nitro-6'-chlorphenyl)-1,4-dihydropyridin-3,5-dicarbonsäurediäthylester gelbe Kristalle vom Fp. 160°C, Ausbeute: 55 %.

e) N-(3'-methoxyphenyl)-2,6-dimethyl-4-(3'-nitrophenyl)-1,4-dihydropyridin-3,5-dicarbonsäurediäthylester Kristalle vom Fp. 136-138°C (hellbeige), Ausbeute: 50 %.

f) N-(2'-methoxyphenyl)-2,6-dimethyl-4-(3'-nitrophenyl)-1,4-dihydropyridin-3,5-dicarbonsäurediäthylester ockergelbe Kristalle vom Fp. 122°C, Ausbeute: 70 %.

g) N-(4'-methoxyphenyl)-2,6-dimethyl-4-thienyl-1,4-dihydropyridin-3,5-dicarbonsäurediäthylester gelbbraune Kristalle vom Fp. 106-108°C, Ausbeute : 70%.

Beispiel 11

N-(4'-methoxyphenyl)-2,6-dimethyl-4-(β-pyridyl)-1,4-dihydropyridin-3-carbonsäureäthyl-5-carbonsäuremethylester

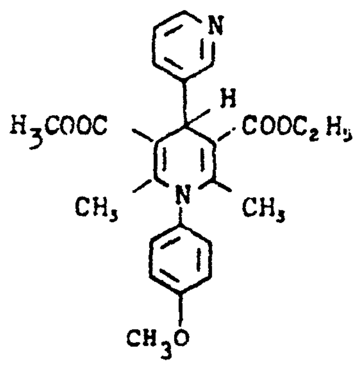

Man erhitzt die Lösung von 10,2 ccm Pyridin-3-aldehyd, 13 ccm Acetessigsäuremethylester und 23,5 g ß-Anisodino-crotonsäure-äthylester (Fp. 45°C) in 80 ccm Aethanol über Nacht am Rückfluss und erhält nach dem Kühlen hellgelbe Kristalle vom Fp. 156°C, Ausbeute 70%.

<u>Beispiel 12</u>

N-(3'-chlor-4'-methoxyphenyl)-2,6-dimethyl-4-phenyl-1,4-dihydropyridin-3,5-dicarbonsäurediäthylester

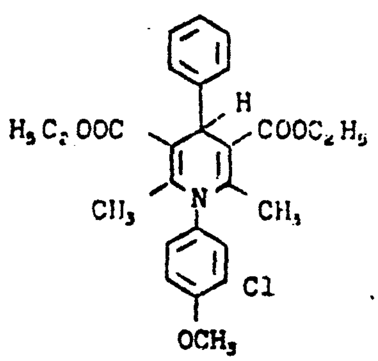

Nach 24 Stdn. erhitzen einer Lösung von 20 ccm Benzaldehyd, 52 ccm Acetessigsäureäthylester und 31,4 g 2-Chlor-4-amino-anisol in 50 ccm Aethanol am Rückfluss werden nach dem Kühlen weisse Kristalle vom Fp. 152°C in 35%iger Ausbeute erhalten.

Auf gleiche Weise wurden erhalten

a) N-(3'-chlor-4'-methoxyphenyl)-2,6-dimethyl-4-(3'-nitrophenyl)-1,4-dihydropyridin-3,5-dicarbonsäurediäthylester, hellgelbe Kristalle vom Fp. 166°C, Ausbeute: 50 %.

b) N-(3'-chlor-4'-methoxyphenyl)-2,6-dimethyl-4-(4'-nitrophenyl)-1,4-dihydropyridin-3,5-dicarbonsäurediäthylester, gelbe Kristalle vom Fp. 141°C, Ausbeute : 45%.

c) N-(3'-chlor-4'-methoxyphenyl)-2,6-dimethyl-4-(3'-nitro-6-chlorphenyl)-1,4-dihydropyridin-3,5-dicarbonsäurediäthylester, ockergelbe Kristalle vom Fp. 173-175°C, Ausbeute : 48%.

d) N-(3'-chlor-4'-methoxyphenyl)-2,6-dimethyl-4-(γ-pyridyl)-1,4-dihydropyridin-3,5-dicarbonsäurediäthylester, weisse Kristalle vom Fp. 176°C, Ausbeute : 40%.

e) N-(3'-chlor-4'-methoxyphenyl)-2,6-dimethyl-4-(2'-chlorphenyl)-1,4-dihydropyridin-3,5-dicarbonsäurediäthylester, hellgelbe Kristalle vom Fp. 150°C, Ausbeute : 25%.

f) N-(3'-chlor-4'-methoxyphenyl)-2,6-dimethyl-4-(4'-chlorphenyl)-1,4-dihydropyridin-3,5-dicarbonsäurediäthylester, Kristalle (hellbeige) vom Fp. 158°C, Ausbeute : 40% .

g) N-(3'-chlor-4'-methoxyphenyl)-2,6-dimethyl-4-(4'-methoxyphenyl)-1,4-dihydropyridin-3,5-dicarbonsäurediäthylester, hellgelbe Kristalle vom Fp. 154°C, Ausbeute : 30 %.

h) N-(3'-chlor-4'-methoxyphenyl)-2,6-dimethyl-4-(2'-4'-dichlorphenyl)-1,4-dihydropyridin-3,5-dicarbonsäurediäthylester, hellgelbe Kristalle vom Fp. 178°C, Ausbeute : 35% .

i) N-(3'-chlor-4'-methoxyphenyl)-2,6-dimethyl-4-(3'-4'-5'-trimethoxyphenyl)-1,4-dihydropyridin-3,5-dicarbonsäurediäthylester, Kristalle (hellbeige) vom Fp. 179°C, Ausbeute : 30%.

j) N-(3'-chlor-4'-methoxyphenyl)-2,6-dimethyl-4-(3'-nitro-4'-methoxyphenyl)-1,4-dihydropyridin-3,5-dicarbonsäurediäthylester, gelbe Kristalle vom Fp. 128°C, Ausbeute : 40%.

k) N-(3'-chlor-4'-methoxyphenyl)-2,6-dimethyl-4-(3'-chlorphenyl)-1,4-dihydropyridin-3,5-dicarbonsäurediäthylester, gelbe Kristalle vom Fp. 146°C, Ausbeute : 40%.

Beispiel 13

N-[4-(γ-dimethyl-amino-propoxy)-phenyl]-2,6-dimethyl-4-(3'-nitrophenyl)-1,4-dihydropyridin-3,5-dicarbonsäurediäthylester

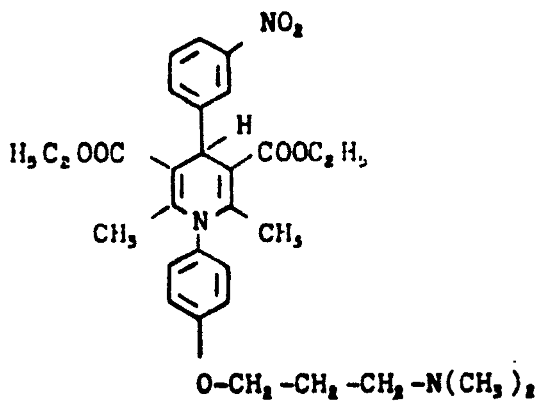

Man gibt zu der warmen Lösung von 46,[illegible] g N-(4'-hydroxyphenyl)-2,6-dimethyl-4-(3'-nitrophenyl)-1,4-dihydropyridin-3,5-dicarbonsäurediäthylester in 150 ccm Aethanol die Lösung von 2,3 g Natrium in 100 ccm Aethanol und tropft unter Sieden langsam 16 ccm γ-Dimethylaminopropylchlorid ein. Nach 5 Stdn. wird heiss abgesaugt und i.V. eingedampft.
Aus Ligroin Kristalle (beige) vom Fp. 116-118°C, Ausbeute : 80%.

Auf gleiche Weise wurden erhalten

a) N-/4'-(β-diäthylaminoäthyl)-phenyl/-2,6-dimethyl-4-(3'-nitrophenyl)-1,4-dihydropyridin-3,5-dicarbonsäurediäthylester, Kristalle (hellbeige) vom Fp. 88-90°C, Ausbeute : 95%.

b) N-4'-(β-diäthylaminoäthyl)-phenyl-2,6-dimethyl-4-(4'-nitrophenyl)-1,4-dihydropyridin-3,5-dicarbonsäurediäthylester, hellgelbe Kristalle (HCl-Salz) Fp. 198°C, Ausbeute: 95 %.

Beispiel 14

N-4-(γ-tert.-butylpylamino-β-hydroxy-propoxy)-phenyl-2,6-dimethyl-4-(4'-nitrophenyl)-1,4-dihydropyridin-3,5-dicarbonsäurediäthylester

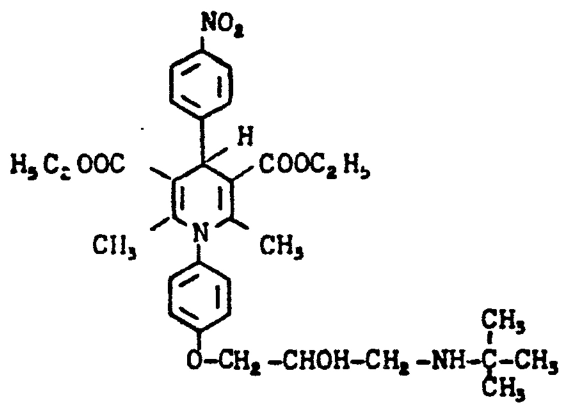

Man erhitzt 10 g N-[4-(β,γ-oxidopropoxy)-phenyl]-2,6-dimethyl-1,4-dihydropyridin-3,5-dicarbonsäurediäthylester (Fp. 165°C) und 4,5 ccm tert.Butylamin in 100 ccm Aethanol über Nacht zum Sieden, engt i.V. ein, versetzt mit Aether und fällt mit ätherischer Salzsäure.
Kristalle (beige) vom Fp. 224°C (HCl-Salz), Ausbeute : 70%.

Auf gleiche Weise wurden dargestellt

a) N-/4-(γ-isopropylamino-β-hydroxy-propoxy)-phenyl/-2,6-dimethyl-4-(4'-nitrophenyl)-1,4-dihydropyridin-3,5-dicarbonsäurediäthylester, Kristalle (beige) vom Fp. 158-160°C (HCl-Salz), Ausbeute: 65 %.

b) N-/4-(γ-isopropylamino-β-hydroxy-propoxy)-phenyl/-2,6-dimethyl-4-(4'-chlorphenyl)-1,4-dihydropyridin-3,5-dicarbonsäurediäthylester, hellgraue Kristalle vom Fp. 180-182°C (HCl-Salz), Ausbeute: 75 %.

Beispiel 15

N-(4'-methoxyphenyl)-2,6-dimethyl-4-(4'-aminophenyl)-1,4-dihydropyridin-3,5-dicarbonsäurediäthylester

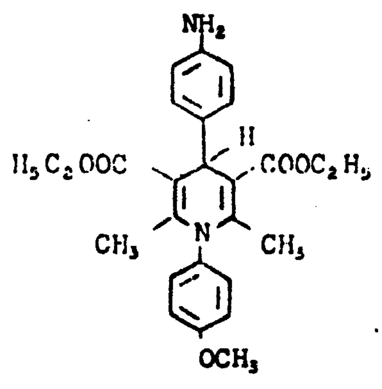

120 g N-(4'-methoxyphenyl)-2,6-dimethyl-4-(4'-nitrophenyl)-1,4-dihydropyridin-3,5-dicarbonsäurediäthylester (Fp. 140°C, Beispiel 9) werden in 1 Liter Aethanol in einem Hochdruck-Rührautoklaven in Gegenwart von 10 g Raney-Ni reduziert. Nach dem Aufarbeiten erhält man weisse Kristalle vom Fp. 130°C in 85 %iger Ausbeute.

a) Auf gleiche Weise wurden aus dem N-(4'-methoxyphenyl)-2,6-dimethyl-4-(3'-nitrophenyl)-1,4-dihydropyridin-3,5-dicarbonsäurediäthylester (Fp. 130°C, Beispiel 9) der N-(4'-methoxyphenyl)-2,6-dimethyl-4-(3'-aminophenyl)-1,4-dihydropyridin-3,5-dicarbonsäurediäthylester in 70 %iger Ausbeute in Kristallen (beige) vom Fp. 170°C und aus dem

b) N-(4'-Methoxyphenyl)-2,6-dimethyl-4-(3'-nitrophenyl)-1,4-dihydropyridin-3,5-dicarbonsäuredi-isopropylester der N-(4'-Methoxyphenyl)-2,6-dimethyl-4-(3'-aminophenyl)-1,4-dihydropyridin-3,5-dicarbonsäuredi-isopropylester in weissen Kristallen vom Fp. 148°C in 80%iger Ausbeute erhalten.

Patentansprüche

1) 1-N-Aryl-1,4-dihydropyridine der allgemeinen Formel (I)

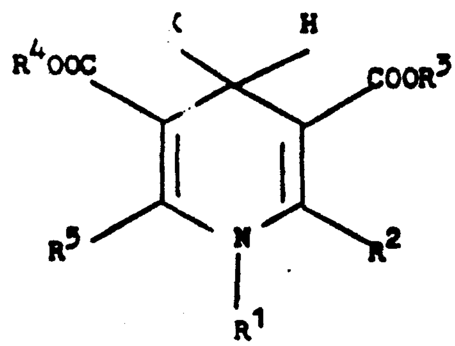

in welcher

$R^1$ für einen Arylrest steht, der gegebenenfalls durch 1, 2 oder 3 gleiche oder verschiedene Substituenten aus der Gruppe Halogen, Hydroxy, Carboxy, Trifluormethyl, Nitro, Cyano, Alkyl, Alkoxy oder den Rest $SO_n$-R', wobei n für O, 1 oder 2 steht und R' gegebenenfalls substituiertes Alkyl bedeutet, substituiert ist, wobei die Alkyl- und Alkoxyreste gegebenenfalls substituiert sind und/oder in der Kette durch Sauerstoff, Stickstoff oder eine COO-Gruppierung unterbrochen sind,

$R^2$ und $R^5$ für gegebenenfalls substituiertes Alkyl oder Alkoxyalkyl stehen,

$R^3$ und $R^4$ gleich oder verschieden sind und jeweils für gegebenenfalls substituiertes Alkyl, Alkenyl oder Alkinyl stehen, wobei die Alkyl- und Alkenyl-Reste gegebenenfalls durch Sauerstoff oder Stickstoff in der Kette unterbrochen sind, und

X für einen gegebenenfalls substituierten Alkylrest steht oder für einen Arylrest steht, der gegebenenfalls durch 1, 2 oder 3 gleiche oder verschiedene Substituenten aus der Gruppe Nitro, Cyano, Azido, Halogen, Trifluormethyl, Trifluormethoxy, Phenyl, Hydroxy, Amino, Alkyl, Alkoxy, Alkoxycarbonyl, Acyloxy, Acylamino, Monoalkylamino, Dialkylamino und $SO_n$-Alkyl substituiert ist, wobei n 0, 1 oder 2 bedeutet oder für gegebenenfalls substituiertes Aralkyl, Styryl, Cycloalkyl, Cycloalkenyl, Chinolyl, Isochinolyl, Pyridyl, Pyrimidyl, Furyl, Thienyl oder Pyrryl steht

sowie ihre pharmakologisch unbedenklichen Salze.

2) Verfahren zur Herstellung von 1-N-Aryl-1,4-dihydropyridinen der allgemeinen Formel (I), dadurch gekennzeichnet, daß man

a) ß-Ketocarbonsäureester der Formel II

$$R^2\text{-CO-CH}_2\text{-COOR}^3 \qquad (II)$$

in welcher

$R^2$ und $R^3$ die oben angegebene Bedeutung besitzen,

mit Aminen oder Formel III

$$H_2N-R^1 \qquad (III)$$

in welcher

$R^1$ die oben angegebene Bedeutung hat

oder deren Salzen gegebenenfalls nach Isolierung der hierbei entstehenden Enamine der Formel IV

$$R^2-\overset{HN-R^1}{\overset{|}{C}}=CH-COOR^3 \quad \text{-}$$

in welcher

$R^1$, $R^2$ und $R^3$ die oben angegebene Bedeutung besitzen,

mit Yliden-Derivaten der Formel V

$$X-CH=\underset{COOR^4}{\underset{|}{C}}-\overset{O}{\overset{\|}{C}}-R^5 \qquad (V)$$

in welcher

X, $R^4$ und $R^5$ die oben angegebene Bedeutung besitzen,

gegebenenfalls in Gegenwart von inerten organischen Lösungsmitteln bei Temperaturen zwischen 20 und 150°C umsetzt,

oder

b) ß-Ketocarbonsäureester der Formel VI

$$R^5-CO-CH_2-COOR^4 \qquad (VI)$$

in welcher

$R^4$ und $R^5$ die oben angegebene Bedeutung besitzen,

mit Aminen der Formel III

$$H_2N-R^1 \qquad (III)$$

in welcher

$R^1$ die oben angegebene Bedeutung hat

oder deren Salzen gegebenenfalls nach Isolierung der hierbei entstehenden Enamine der Formel VII

$$R^5-\underset{}{\overset{NH-R_1}{C}}=CH-COOR^4 \qquad (VII)$$

in welcher

$R^1$, $R^4$ und $R^5$ die oben angegebene Bedeutung besitzen,

mit Yliden-Derivaten der Formel VIII

$$X-CH=\underset{COOR^3}{C}-CO-R^2 \qquad (VIII)$$

in welcher

$R^2$, $R^3$ und X die oben angegebene Bedeutung besitzen,

gegebenenfalls in Gegenwart von inerten organischen Lösungsmitteln bei Temperaturen zwischen 20 und 150°C umsetzt,

oder

c) β-Ketocarbonsäureester der Formel VI

$$R^5\text{-CO-CH}_2\text{-COOR}^4 \qquad (VI)$$

in welcher
$R^4$ und $R^5$ die oben angegebene Bedeutung besitzen,
und Enamine der Formel IV

$$R^2\text{-}\underset{}{\overset{NH\text{-}R^1}{C}}\text{=CH-COOR}^3 \qquad (IV)$$

in welcher

$R^1$, $R^2$ und $R^3$ die oben angegebene Bedeutung besitzen,

mit Aldehyden der Formel IX

$$X\text{-CHO} \qquad (IX)$$

in welcher

X die oben angegebene Bedeutung hat,

gegebenenfalls in Gegenwart von inerten organischen Lösungsmitteln bei Temperaturen zwischen 20 und 150°C umsetzt,

oder

d) β-Ketocarbonsäureester der Formel II

$$R^2\text{-CO-CH}_2\text{-COOR}^3 \qquad (II)$$

in welcher

$R^2$ und $R^3$ die oben angegebene Bedeutung besitzen,

mit Enamine der Formel VII

$$R^5\text{-}\overset{\displaystyle NH\text{-}R^1}{\overset{|}{C}}\text{=CH-COOR}^4 \qquad (VII)$$

in welcher

$R^1$, $R^4$ und $R^5$ die oben angegebene Bedeutung besitzen,

mit Aldehyden der Formel IX

$$X\text{-CHO} \qquad (IX)$$

in welcher

X die oben angegebene Bedeutung besitzt,

gegebenenfalls in Gegenwart von inerten organischen Lösungsmittel bei Temperaturen zwischen 20 und 150°C umsetzt,

oder

e) falls $R^2$ mit $R^3$ und $R^5$ mit $R^4$ identisch sind, zwei Teile β-Ketocarbonsäureester der Formel VI

$$R^5\text{-CO-CH}_2\text{-COOR}^4 \qquad (VI)$$

in welcher

$R^4$ und $R^5$ die oben angegebene Bedeutung besitzen,

mit einem Teil Amin der Formel III

$$H_2N\text{-R} \qquad (III)$$

in welcher

$R^1$ die oben angegebene Bedeutung besitzt,

oder dessen Salz mit einem Teil Aldehyd der Formel IX

$$X\text{-CHO} \qquad (IX)$$

in welcher

X die oben angegebene Bedeutung besitzt,

gegebenenfalls in Gegenwart von inerten organischen Lösungsmitteln bei Temperaturen zwischen 20 und 150°C umsetzt.

3) 1-N-Aryl-1,4-dihydropyridine gemäß der Formel (I) in Anspruch 1, in welcher

$R^1$ für einen Phenylrest, der durch 1, 2 oder 3 gleiche oder verschiedene Substituenten aus der Gruppe Halogen, insbesondere Fluor, Chlor oder Brom, Hydroxy, Carboxy, Trifluormethyl, Nitro, Cyano, Alkyl, Alkoxy, Alkylmercapto, Alkylaminoalkyl und Dialkylaminoalkyl substituiert ist, steht, wobei die vorgenannten Alkyl- und Alkoxygruppen je 1 bis 4 Kohlenstoffatome enthalten und gegebenenfalls noch durch ein Sauerstoffatom in der Kette oder in der Anknüpfungsstelle zum Phenylring unterbrochen sind,

$R^2$ und $R^5$ gleich oder verschieden sind und für Wasserstoff oder Alkyl mit 1 bis 4 Kohlenstoffatomen stehen, wobei die Alkylkette gegebenenfalls durch ein Sauerstoffatom unterbrochen ist,

$R^3$ und $R^4$ gleich oder verschieden sind und für Alkyl, Alkenyl oder Alkinyl mit bis zu 6, insbesondere mit bis zu 4 Kohlenstoffatomen stehen, wobei die Alkyl- und Alkenylketten gegebenenfalls durch ein Sauerstoff- oder Stickstoffatom in der Kette unterbrochen sind und

X für einen Alkylrest mit 1 bis 4 Kohlenstoffatomen steht oder für einen Phenylrest steht, der gegebenenfalls durch 1 oder 2 Substituenten aus der Gruppe Nitro, Halogen, insbesondere Fluor, Chlor oder Brom, Trifluormethyl, Amino,

Alkylamino, Dialkylamino, Alkylalkoxy, Alkoxyalky, Alkylmercapto, Alkenyl und Alkinyl substituiert ist, wobei die genannten Alkyl-, Alkoxy-, Alkenyl- und Alkinyl-Reste jeweils bis zu 4 Kohlenstoffatome enthalten
oder
für einen gegebenenfalls durch Halogen, Nitro, Trifluormethyl, Alkoxy oder Alkyl mit je 1 oder 2 Kohlenstoffatomen substituierten Pyridylrest, Thienylrest, Naphthylrest oder Chinolylrest.

4) Arzneimittel enthaltend mindestens ein 1-N-Aryl-1,4-dihydropyridin gemäß Anspruch 1.

5) Verfahren zur Herstellung von Arzneimitteln, dadurch gekennzeichnet, daß man 1-N-Aryl-1,4-dihydropyridine gemäß Anspruch 1 gegebenenfalls unter Zusatz von inerten pharmazeutisch unbedenklichen Hilfs- und Trägerstoffen in eine geeignete Applikationsform überführt.

6) Verwendung von 1-N-Aryl-1,4-dihydropyridinen gemäß Anspruch 1 bei der Bekämpfung von Erkrankungen.

7) Verfahren zur Behandlung von Kreislauferkrankungen, dadurch gekennzeichnet, daß man 1-N-Aryl-1,4-dihydropyridine gemäß Anspruch 1 Menschen oder Tieren im Bedarfsfall appliziert.

EUROPÄISCHER RECHERCHENBERICHT

EINSCHLÄGIGE DOKUMENTE

| Kategorie | Kennzeichnung des Dokuments mit Angabe, soweit erforderlich, der maßgeblichen Teile | betrifft Anspruch |
| --- | --- | --- |
| X | JOURNAL OF MEDICINAL CHEMISTRY, ... 1966 ... et al. "Hantzsch-Type Dihydropyridine potentive agents" * Seite 959, Beispiele 44,47; Seite 962; Seite 964, rechte Spalte, Absatz 5 * | 1, 2, 4-7 |
| | -- A - 2 616 995 (BAYER) * Patentansprüche * | 1, 2, 4-7 |
| | -- A - 2 616 991 (BAYER) * Patentansprüche * | 1, 2, 4-7 |
| | -- A - 2 658 804 (BAYER) Patentansprüche; Seiten 7-9 * | 1, 2, 4-7 |

KLASSIFIKATION DER ANMELDUNG: C 07 D ... A 61 K ...

RECHERCHIERTE SACHGEBIETE: C 07 D 211/90 ... A 61 K 31/44

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
| --- | --- | --- |
| Den Haag | 12-02-1979 | NUYTS |

EPA form 1503.1 06.78